# EUROPÄISCHE PATENTSCHRIFT

(11) **EP 1 158 897 B1**
(45) Veröffentlichungstag und Bekanntmachung des Hinweises auf die Patenterteilung: **26.01.2005**
(21) Anmeldenummer: 00916909.5
(22) Anmeldetag: 08.03.2000
(51) Int. Cl.: A61B 5/22

(54) **VERFAHREN ZUR BESTIMMUNG DER BELASTBARKEIT EINES MENSCHEN**
METHOD FOR DETERMINING THE STRESS CAPACITY OF A PERSON
PROCEDE POUR DETERMINER LA CAPACITE DE CHARGE D'UN ETRE HUMAIN

(30) Priorität: 08.03.1999 DE 19909852
(43) Veröffentlichungstag der Anmeldung: 05.12.2001
(73) Patentinhaber: Stegmann, Heiner, 63450 Hanau (DE)
(72) Erfinder: Stegmann, Heiner, 63450 Hanau (DE)
(74) Vertreter: Stoffregen, Hans-Herbert, Dr. Dipl.-Phys.
(86) Internationale Anmeldenummer: PCT/EP2000/002030
(87) Internationale Veröffentlichungsnummer: WO 2000/053091

(56) Entgegenhaltungen:
- US-A- 4 622 980

## Beschreibung

Die Erfindung bezieht sich auf Verfahren zum Bestimmen der Belastbarkeit eines Menschen unter Berücksichtigung der individuellen anaeroben Schwelle durch nicht invasives Messen von Laktat-Konzentrationen in Abhängigkeit von geleisteter Arbeit.

Die Laktat-Konzentration des Blutplasmas wird in der Belastungsphase überwiegend durch den Laktat-Influx bestimmt, d. h. durch den überwiegend diffusiven Laktatfluss vom Muskel ins Blutplasma. Dieser Influx wird angetrieben durch das bei Belastung auftretende Konzentrationsgefälle zwischen Laktat produzierendem Muskelgewebe und Blutplasma. In der Nachbelastungsphase dagegen überwiegt der Einfluss des Laktat-Efflux, d. h. der Laktatelimination aus dem Blutplasma.

Aus der Laktat-Konzentration kann die sogenannte individuelle anaerobe Schwelle bestimmt werden, bei der es sich um einen festen, eindeutig reproduzierbaren Wert handelt. Dabei ist die "individuelle anaerobe Schwelle" auch für physiologische Parameter wie Beurteilung der körperlichen Leistungsfähigkeit, Belastungsblutdruck und koronare Herzerkrankungen von Bedeutung.

Üblicherweise wird die individuelle anaerobe Schwelle durch Milchsäurebestimmung im Blut vorgenommen, wobei die Änderungen des Milchsäureanteils in Abhängigkeit von der pro Zeiteinheit geleisteten Arbeit gemessen wird.

In der Literatur hat sich die Bestimmung der individuellen anaeroben Schwelle nach Stegmann et al. (Int. J. Sports Medicine 2 (1981) 160 - 165) bei der leistungsphysiologischen Diagnostik durchgesetzt und breite Anwendung gefunden. Die individuelle anaerobe Schwellenbestimmung nach Stegmann unterscheidet sich von anderen Laktatschwellenkonzepten durch die Berücksichtigung des Plasma-Laktatspiegels sowohl in der Belastungsphase als auch in der Nachbelastungsphase. In der Nachbelastungsphase überwiegt der Einfluss des Laktat-Efflux, d.h. die Laktatelimination aus dem Blutplasma. Die Messung der Laktatkinetik über das Belastungsende hinaus und die Verwendung dieser Kinetik in der Herleitung der individuellen anaeroben Schwelle bietet die Gewähr, dass bei der Bestimmung der individuellen anaeroben Schwellen sowohl Parameter der Laktat-Influx- als auch der Laktat-Efflux-Charakteristik des unter Belastung stehenden Probanden mitberücksichtigt werden. Aus dieser Berücksichtigung leitet sich ein großer Vorteil der Stegmann-Schwelle ab, da die Bestimmung der individuellen anaeroben Schwellen im Belastungstest mit beliebigen festgewählten Stufen dauernd durchgeführt werden kann.

Die individuelle anaerobe Schwelle nach Stegmann lässt sich jedoch nicht nur durch fortwährende Laktatmessung aus dem Blut des Menschen, sondern auch aus den respiratorischen Messgrößen des Atem-Minutenvolumens, des O₂-Gehalts des Atem-Minutenvolumens sowie dem CO₂-Gehalt des Atem-Minutenvolumens bestimmen (EP 0 742 693 B1).

Der vorliegenden Erfindung liegt das Problem zu Grunde, ein Verfahren der eingangs genannten Art so weiterzubilden, dass die Möglichkeit besteht, aus der Laktat-Konzentrationsänderung weitere für den Menschen charakteristische Leistungsdaten zu gewinnen.

Erfindungsgemäß wird das Problem durch die Verfahrensschritte gelöst
- Messung der zeitabhängigen Laktat-Konzentrationsänderung über die individuelle anaerobe Schwelle hinaus,
- Anpassen einer Messkurve an so gewonnene Messwerte, in der die Laktatkonzentration gegenüber der Zeit aufgetragen wird,
- Bestimmung einer ersten Steigung der Messkurve in einem der individuellen anaeroben Schwelle entsprechenden Zeitpunkt t_{IAT},
- Bestimmung zumindest einer weiteren Steigung aus der Messkurve zu einem - Zeitpunkt tₓ mit tₓ > t_{IAT} und
- Subtraktion der zweiten Steigung von der ersten Steigung zur Bestimmung einer Differenz ΔA.

Entsprechend abgespeicherte Kurven bzw. aus diesen ermittelte Differenzen ΔA können mit zu anderen Zeiten aufgenommenen Werten desselben Menschen oder mit Messkurven unterschiedlicher Menschen oder mit Standardwerten verglichen werden, um auf diese Weise Kenndaten der Leistungsfähigkeit zu gewinnen. Die Messkurven selbst können bei stufenweiser oder kontinuierlicher Steigerung der Belastung des Menschen aufgenommen werden.

Erfindungsgemäß benutzt man die Kenntnis, dass bei einer Belastungssteigerung über die individuelle anaerobe Schwelle (IAT) hinaus ein starker Anstieg der Laktatkonzentration im Blutplasma erfolgt; da durch das Überwiegen des Laktat-Influx gegenüber dem Laktat-Efflux im Plasmakompartiment Laktat akkumuliert. Wird die Steigung der Laktat-Konzentrations-Zeit-Kurve, die auch als Laktatakkumulationsrate zu bezeichnen ist, bei Erreichen der individuellen anaeroben Schwelle (IAT) durch den Zustand eines Organismus charakterisiert, bei dem eine Dauerbelastung mit der der individuellen anaeroben Schwelle entsprechenden Leistung nicht zu einem wachsenden Anstieg, sondern zu einem Sistieren der Laktatkonzentrations-Zeit-Kurve auf maximalem Niveau führt, so ist dann, wenn die der individuellen anaeroben Schwelle entsprechende Leistung überschritten wird, ein rascher Anstieg der Laktatkonzentration im Blutplasma festzustellen.

Erfindungsgemäß erfolgt eine Normierung der Laktat-Akkumulationsrate auf die der individuellen anaeroben Schwelle mit der Folge, dass charakteristische Leistungswerte des Menschen zur Verfügung stehen.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung nachstehender Figuren.

Es zeigen:
- Fig. 1: eine Laktat-Leistungs-Kurve,
- Fig. 2: eine der Laktat-Leistungs-Kurve gemäß Fig. 1 zugeordnete Leistungs-Zeit-Kurve bei stufenförmiger Leistungsänderung,
- Fig. 3: eine der Laktat-Leistungs-Kurve gemäß Fig. 1 zugeordnete Leistungs-Zeit-Kurve bei kontinuierlicher Leistungsänderungen und
- Fig. 4: Laktat-Akkumulationsratenänderungen in Abhängigkeit von der Leistung.

Um charakteristische reproduzierbare Werte über die Leistungsfähigkeit bzw. Belastbarkeit eines Menschen zu gewinnen, wird erfindungsgemäß die Änderung der Laktatkonzentration in Abhängigkeit von der zeitlichen Belastung gemessen. Zur Belastungsmessung kann dabei ein Fahrradergometer oder zum Beispiel ein Laufband oder ein sonstiges Gerät oder eine Anordnung benutzt werden, das auch bei der Bestimmung der individuellen anaeroben Schwelle (IAT) Anwendung findet. Die Geräte sind über Leitungen mit einer Datenverarbeitungsanlage verbunden, um so die pro Zeit-Einheit geleistete Arbeit zu ermitteln. Des Weiteren erfolgt zeitabhängig eine Überprüfung vom Blut des Menschen. Die entsprechenden Blurwerte, d.h. deren für die Laktatkonzentration charakteristischen Werte wie Milchsäureanteil werden der Datenverarbeitungsanlage ebenfalls zugeführt.

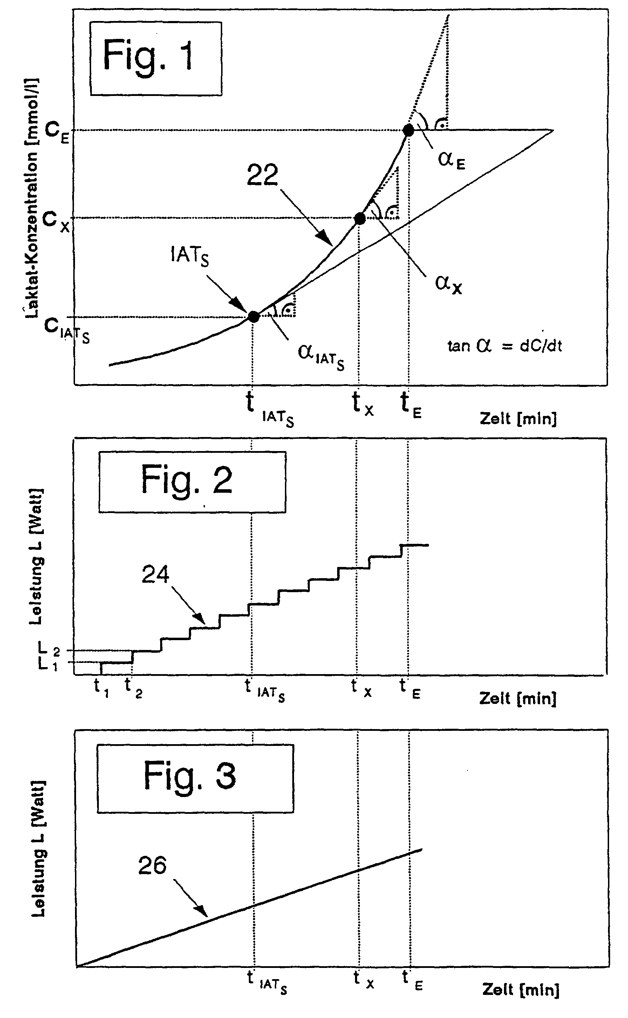

Aus den so gewonnenen Werten berechnet die Datenverarbeitungsanlage eine Messkurve, in der entsprechend der Fig. 1 die Konzentration C des Laktats in mmol gegenüber der Zeit t vorzugsweise in Minuten aufgetragen ist. Die entsprechende Messkurve ist in Fig. 1 mit dem Bezugszeichen 22 versehen.

Wie aus den Fig. 2 und 3 ersichtlich, kann die Belastungssteigerung stufenweise (Kurve 24) oder kontinuierlich (Kurve 26) erfolgen. Insoweit wird jedoch auf hinreichend bekannte Messverfahren verwiesen.

Die Konzentrationsänderung des Laktats wird erfindungsgemäß nicht nur bis zu der anaeroben individuellen Schwelle IAT, also über die Dauerbelastungsgrenze hinaus, sondern auch bei größerer Belastung bestimmt, bei der ein rascher Anstieg der Laktatkonzentration im Blutplasma feststellbar ist. Auch bei den größeren Belastungen erfolgt eine stufenweise (Fig. 2) oder kontinuierliche Belastungssteigerung (Fig. 3), also in analoger Weise, wie dies von der Bestimmung der individuellen Schwelle IAT bekannt ist. Aus der Messkurve 22 werden sodann die Steigungen der Laktat-Konzentrationszeitkurve sowohl in dem der individuellen anaeroben Schwelle IAT entsprechenden Zeitpunkt t_{IAT} als auch in weiteren Zeitpunkten wie tₓ > t_{IAT} und gegebenenfalls bis zum Belastungsende t_{E} bestimmt.

Die entsprechenden Steigungen können als Laktat-Akkumulationsrate gewertet werden, wobei erkennbar die Laktat-Akkumulationsraten oberhalb des Zeitpunktes t_{IAT} zunehmen, also die Steigung tanα_{IAT} zu einem Zeitpunkt t_{IAT} kleiner als die Steigung tanαₓ zu einem Zeitpunkt tₓ > t_{IAT} ist, die wiederum kleiner als die Steigung tα_{E} zu einem Zeitpunkt t_{E} am Belastungsende ist.

Aus der Laktatleistungskurve 22 oberhalb der individuellen anaeroben Schwelle IAT können sodann charakteristische reproduzierbare Werte des belasteten Menschen gewonnen werden, wenn eine Bereinigung der Laktat-Akkumulationsrate um die der individuellen anaeroben Schwelle erfolgt, also die Differenz ΔA zwischen der Steigung tanαₓ und der Steigung tanα_{IAT} gebildet wird. Diese Differenz ist ein Maß für die Systemstörung der Laktatkonzentration oberhalb der der individuellen anaeroben Schwelle entsprechenden Dauerbelastung und somit für die jeweiligen Menschen individuell charakteristisch.

Die Leistungsfähigkeit bzw. Belastbarkeit des Menschen kann sowohl aus der der Fig. 1 zu entnehmenden Laktatleistungskurve als auch einer Kennlinie 28 entnommen werden, bei der die Änderung der Laktat-Akkumulationsrate ΔA gegenüber der Leistung L aufgetragen wird. Die Kennlinie 28 kann dabei mit einer Normkurve 30, mit einer von demselben Menschen zu einem anderen Zeitpunkt aufgenommenen Kurve oder mit der Messkurve eines anderen Menschen verglichen werden, um somit Aussagen über die Leistungsfähigkeit bzw. Belastbarkeit und gegebenenfalls normierte Werte abzuleiten.

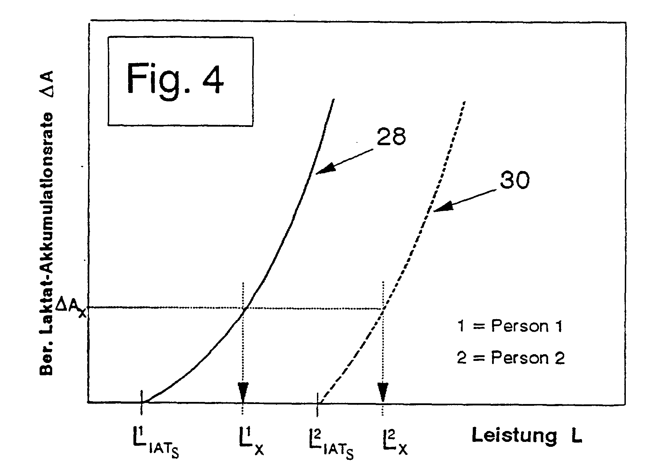

Aus der Fig. 4 erkennt man die Korrelation zwischen unterschiedlichen Leistungen L¹ₓ und L²ₓ, die bei gleicher um die individuelle aerobe Schwelle bereinigte Laktat-Akkumulationsrate ΔA im Ausbelastungstest erbracht werden.

Somit ergibt sich aus der Bestimmung der um die individuelle anaerobe Schwelle bereinigten Laktat-Akkumulationsrate ΔA im Belastungstest ein Rückschluss auf die Ausbelastungsdauer eines Menschen. Dies wiederum bedeutet, dass die bereinigte Laktat-Akkumulationsrate ΔA als Parameter zur allgemeinen Trainingssteuerung verwendet werden kann. Auch können die um die individuelle anaerobe Schwelle bereinigten Laktat-Akkumulationsraten ΔA mit der Zielrichtung ausgewertet werden, koronare Herzkrankheiten zu erkennen oder Blutdruckregulierungsvorgänge zu beurteilen.

## Patentansprüche

1. Verfahren zum Bestimmen der Belastbarkeit eines Menschen unter Berücksichtigung der individuellen anaeroben Schwelle durch nicht invasives Messen von Laktat-Konzentrationen in Abhängigkeit von geleisteter Arbeit, mit den Verfahrensschritten:
- Messung der zeitabhängigen Laktat-Konzentrationsänderung über die individuelle anaerobe Schwelle hinaus,
- Anpassen einer Messkurve an so gewonnene Messwerte, in der die Laktatkonzentration gegenüber der Zeit aufgetragen wird,
- Bestimmung einer ersten Steigung der Messkurve in einem der individuellen anaeroben Schwelle entsprechenden Zeitpunkt t_{IAT},
- Bestimmung zumindest einer weiteren Steigung aus der Messkurve zu einem Zeitpunkt tₓ mit tₓ > t_{IAT} und
- Subtraktion der zweiten Steigung von der ersten Steigung zur Bestimmung einer Differenz ΔA.

2. Verfahren nach Anspruch 1,
**dadurch gekennzeichnet,**
**dass** Differenzen ΔAₓ mit x = 1,2... gegenüber der Leistung aufgetragen werden und so gebildete Messkurven mit Normmesskurven, Messkurven unterschiedlicher Menschen oder Messkurven desselben Menschen zu unterschiedlichen Belastungszeiten verglichen werden.

3. Verfahren nach Anspruch 1 oder 2,
**dadurch gekennzeichnet,**
**dass** die Messkurven bei stufenweiser Leistungsänderung des Menschen gemessen werden.

4. Verfahren nach Anspruch 1 order 2,
**dadurch gekennzeichnet,**
**dass** die Messkurven bei kontinuierlicher Leistungsänderung des Menschen gemessen werden.

5. Verfahren nach zumindest einem der vorhergehenden Ansprüche,
**dadurch gekennzeichnet,**
**dass** als individuelle anaerobe Schwelle die nach Stegmann bestimmte als Subtraktionsparameter zur Bestimmung der Differenz ΔA benutzt wird.

## Claims

1. Process for determining the stress capacity of a person while taking into consideration the individual anaerobic threshold by non-invasive measuring of lactate concentrations in dependence upon the work performed, with the process steps:
- measuring the time-depended lactate concentration changes above the individual anaerobic threshold,
- adjusting a measured curve to the obtained measured values in which the lactate concentration is recorded with respect to time,
- determining a first rise in the measured curve at a time point t_{IAT} corresponding to the individual anaerobic threshold,
- determining at least one more rise from the measured curve at a time point t_{X}, with t_{X} > t_{IAT}, and
- subtracting the second rise from the first rise for determining a difference ΔA.

2. Process according to claim 1,
**characterized in**
**that** the differences ΔAₓ with x = 1, 2... are plotted against the effort and the measured curves formed in this way are compared with standard curves, measured curves of different persons, or measured curves of the same person at different stress times.

3. Process according to claim 1 or 2,
**characterized in**
**that** the measured curves are measured during a staged effort change of the person.

4. Process according to claim 1 or 2,
**characterized in**
the measured curves are measured during a continuous effort change of the person.

5. Process according to at least one of the preceding claims,
**characterized in**
**that** as individual anaerobic threshold the one determined by Stegmann is used as subtraction parameter for determining the difference ΔA.

## Revendications

1. Procédé pour déterminer la résistance d'un individu en tenant compte du seuil anaérobie individuel par mesure non traumatique des concentrations de lactate en fonction du travail fourni, comprenant les étapes suivantes :
- mesure de la modification de la concentration de lactate en fonction du temps, au-delà du seuil anaérobie,
- adaptation d'une courbe de mesure aux valeurs mesurées ainsi acquises, dans laquelle la concentration de lactate est portée par rapport au temps,
- détermination d'une première pente de la courbe de mesure dans un instant t_{IAt} correspondant au seuil anaérobie individuel,
- détermination d'au moins une autre pente sur la courbe de mesure à instant tₓ, sachant que tₓ > t_{IAt} et
- soustraction de la deuxième pente de la première pente pour déterminer une différence ΔA.

2. Procédé selon la revendication 1,
c**aractérisé en ce**
que des différences ΔAₓ avec x = 1, 2, ... sont portées en fonction du travail, et que les courbes de mesure ainsi formées sont comparées avec des courbes de mesure normées, des courbes de mesure de différents individus ou des courbes de mesure du même individus pour différents temps d'effort.

3. Procédé selon la revendication 1 ou 2,
**caractérisé en ce**
**que** les courbes de mesure sont mesurées lors d'une modification par paliers du travail de l'individu.

4. Procédé selon la revendication 1 ou 2,
**caractérisé en ce**
**que** les courbes de mesure sont mesurées lors d'une modification continue du travail de l'individu.

5. Procédé selon l'une de revendications précédentes,
**caractérisé en ce**
**qu'**on utilise comme seuil anaérobie individuel le seuil défini selon Stegmann comme paramètre de soustraction pour déterminer la différence ΔA.
